# EUROPEAN PATENT SPECIFICATION

(11) **EP 4 427 682 B1**
(45) Date of publication and mention of the grant of the patent: **14.10.2026**
(21) Application number: 24159176.7
(22) Date of filing: 22.02.2024
(51) Int. Cl.: A61B 17/12, A61B 17/00

(54) **OCCLUSION DEVICE**
OKKLUSIONSVORRICHTUNG
DISPOSITIF D'OCCLUSION

(30) Priority: 07.03.2023 US 202363488822 P; 01.12.2023 US 202318526916
(43) Date of publication of application: 11.09.2024
(73) Proprietor: Medtronic, Inc., Minneapolis, MN 55432 (US)
(72) Inventor: JOSAITIS, James, Minneapolis, 55432 (US); SCHOCH, David, Minneapolis, 55432 (US); MULLEN, Eithne, Minneapolis, 55432 (US)
(74) Representative: Zimmermann & Partner Patentanwälte mbB

(56) References cited:
- WO-A1-2023/009662
- CN-A- 101 304 693
- CN-A- 105 208 943
- US-A1- 2008 269 720
- US-A1- 2012 065 662

## Description

### CROSS-REFERENCE TO RELATED APPLICATIONS

### FIELD

The present teachings relate to an occlusion device for occluding a left atrial appendage of a heart.

### BACKGROUND

The human heart is a four chambered, muscular organ that provides blood circulation through the body during a cardiac cycle. The four main chambers include the right atrium RA and right ventricle RV which supplies the pulmonary circulation, and the left atrium LA and left ventricle LV which supplies oxygenated blood received from the lungs into systemic circulation. To ensure that blood flows in one direction through the heart, atrioventricular valves (tricuspid valves TV and mitral valves MV) are present between the junctions of the atria and the ventricles, and semi-lunar valves (pulmonary valve and aortic valve) govern the exits of the ventricles leading to the lungs and the rest of the body. The heart also includes a left atrial appendage LAA, which is a small, ear-shaped sac in the muscle wall of the left atrium LA. In normal hearts, when the heart contracts, the blood in the left atrium LA and the left atrial appendage LAA is squeezed out of the left atrium LA and into the left ventricle LV.

In atrial fibrillation, an irregular heartbeat causes blood flow to slow enabling clots to form. Because the left atrial appendage LAA is a small sac or pouch, blood may collect there and form clots. When blood clots are pumped out of the heart, they can cause a stroke. Thus, in some cases, it may be desirable to exclude or occlude the left atrial appendage LAA such that clots do not form in the left atrial appendage LAA, and if they do, they cannot escape the left atrial appendage LAA.

WO 2023/009662 A1 describes methods, systems, and devices for occluding the left atrial appendage of a patient's heart. US 2012/065662 A1 describes a device and method for obliterating or occluding a body cavity or passageway, in particular, the left atrial appendage of a patient's heart. The occluding device may prevent the passage of embolic or other material to or from the left atrial appendage by volumetrically filling the appendage, closing the opening of the appendage with an occluding member, or pulling the tissue around the opening of the appendage together and fixing it in a closed state.

The present disclosure relates to improvements in catheter-based occlusion systems for occluding or excluding the left atrial appendage.

### SUMMARY

A first aspect of the invention provides an occlusion device for occluding a left atrial appendage, the device comprising: a body; at least one anchor connected to the body and moveable between a delivery configuration and a deployed configuration in which the at least one anchor is configured to anchor the occlusion device in the left atrial appendage; and an occlusion assembly connected to the body, wherein the occlusion assembly comprises a plurality of elongate occluding filaments extending in a direction away from the at least one anchor, wherein the plurality of elongate occluding filaments are arranged in a cluster configuration, wherein each of the plurality of elongate occluding filaments is configured to move from a radially compressed configuration into a radially expanded configuration for occluding and sealing the left atrial appendage, and wherein, in the radially expanded configuration, the occlusion assembly is configured to be oversized relative to an opening of the left atrial appendage, wherein, due to the occlusion assembly being oversized relative to the opening of the left atrial appendage, the left atrial appendage will apply an external force to the plurality of elongate occluding filaments to cause the plurality of elongate occluding filaments to inter-engage and/or inter-mesh to occlude the left atrial appendage.

In some embodiments, the plurality of elongate occluding filaments may be configured and arranged to inter-engage and/or inter-mesh upon application of an external force for preventing a thrombus from passing therethrough.

In some embodiments, each of the plurality of elongate occluding filaments may be configured to swell and/or radially expand to move into the radially expanded configuration. In some embodiments, in the radially expanded configuration, the plurality of elongate occluding filaments may comprise one or more of: spiral filaments; cylindrical filaments; tubular filaments; and/or conical filaments. In some embodiments, each of the plurality of elongate occluding filaments may be configured to be radially compressible in the radially expanded configuration.

In some embodiments, each of the plurality of elongate occluding filaments may comprise a self-expanding material. In some embodiments, each of the plurality of elongate occluding filaments may comprise a sponge material, a shape memory material such as a shape memory polymer or nitinol, and/or a polymeric material.

In some embodiments, the occlusion assembly may comprise a core and wherein the plurality of elongate occluding filaments are arranged around said core. In some embodiments, the core may be formed from a self-expanding material. In some embodiments, the core may be formed from a shape memory material such as a shape memory polymer or nitinol. In some embodiments, the core may be substantially rigid.

In some embodiments, the at least one anchor is configured to move into the deployed configuration upon deployment from a catheter body. In some embodiments, the at least one anchor may be formed from a self-expanding material. In some embodiments, the at least one anchor may be formed from a shape memory material, for example nitinol.

In some embodiments, the occlusion device may comprise an actuating arrangement to move the at least one anchor into the deployed configuration.

In some embodiments, the body may comprise a chamber containing a filler material and an aperture fluidly coupled to the chamber for dispensing the filler material.

According to a second aspect of the invention, there is provided a delivery catheter system comprising: a catheter assembly comprising an elongate catheter body having a distal tip portion; and an occlusion device as described herein according to the invention positioned within the catheter body, wherein the occlusion assembly is arranged such that the at least one anchor is positioned distally relative to the occlusion assembly, wherein the occlusion device is deployable from the distal tip portion of the catheter body so as to enable the at least one anchor to move from the delivery configuration to the deployed configuration, and wherein the plurality of elongate occluding filaments are configured to move from the radially compressed configuration to the radially expanded configuration upon deployment from the catheter body.

In some embodiments, the delivery catheter assembly may comprise a delivery member configured and arranged to deliver a filler material to a position distal to the occlusion assembly.

In some embodiments, the delivery member may be positioned within the catheter body.

In some embodiments, the delivery member may be arranged to extend through or adjacent to the plurality of elongate filaments.

### BRIEF DESCRIPTION OF THE DRAWINGS

Embodiments will now be described with reference to the accompanying drawings, in which:
Figure 1 is a schematic sectional illustration of a heart;
Figure 2 is a schematic view of a delivery catheter system in a first configuration according to an embodiment;
Figure 3 is a schematic view of the delivery catheter system of Figure 2 in a second configuration;
Figure 4 is a schematic view of an occlusion device according to an embodiment;
Figure 5 is a schematic view of the occlusion device of Figure 4 in a left atrial appendage;
Figure 6 is a schematic view of a delivery catheter system in a first configuration according to an embodiment;
Figure 7 is a schematic view of the delivery catheter system of Figure 6 in a second configuration;
Figure 8 is a schematic view of an occlusion device having an occlusion assembly in a delivery configuration according to an embodiment ;
Figure 9 is a schematic view of the occlusion device of Figure 7 with the occlusion assembly in a deployed configuration;
Figures 10A-D are schematic views showing a trans-septal approach to the left atrial appendage according to an embodiment; and
Figures 11 to 13 are schematic views of deploying an occlusion device in a left atrial appendage according to an embodiment.

### DETAILED DESCRIPTION OF EMBODIMENT(S)

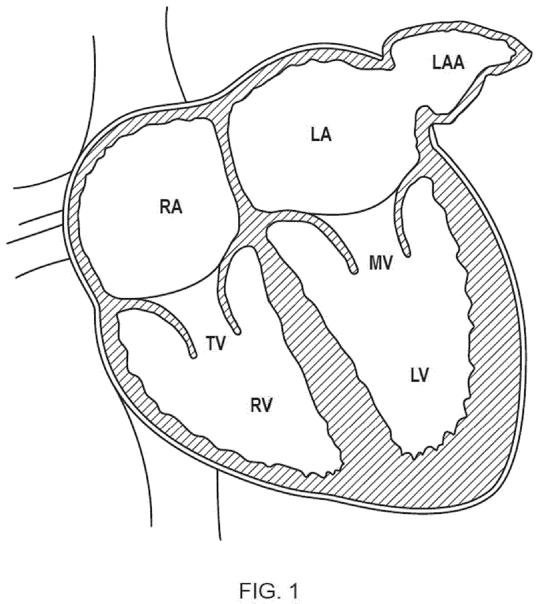

Figure 1 is a schematic sectional illustration of a human heart that depicts the four heart chambers (right atrium RA, right ventricle RV, left atrium LA, left ventricle LV) and a left atrial appendage LAA.

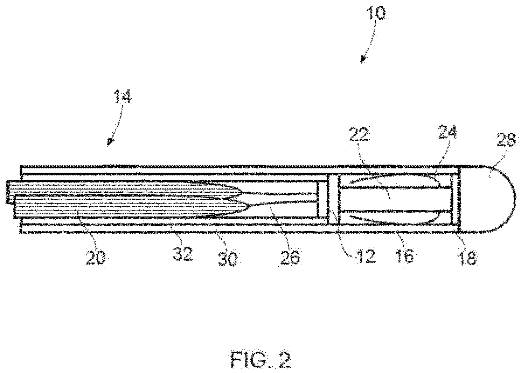

Referring to Figure 2, a delivery catheter system 10 is illustrated. The delivery catheter system 10 is configured for delivering and deploying an occlusion device 12 within the left atrial appendage of a patient.

The delivery catheter system 10 has a catheter assembly 14 including an elongate catheter body 16 having a distal tip portion 18. The occlusion device 12 is at least partially, for example entirely, positioned within the catheter body 16. When the occlusion device 12 is contained within the catheter body 16, as is illustrated in figure 2, the occlusion device 12 is in a delivery configuration. The delivery configuration may be considered to be one in which at least a part of the occlusion device is in a radially compressed configuration.

The occlusion device 12 includes an occlusion assembly 20. In the delivery configuration, the occlusion assembly 20 is in a radially compressed configuration. Put another way, in the delivery configuration, the occlusion assembly 20 has a reduced cross-sectional diameter compared to when the occlusion assembly 20 is in a deployed configuration, or radially expanded configuration. This configuration of the occlusion device, and in particular the occlusion assembly, reduces the diameter of the occlusion assembly to enable the occlusion device to the delivered to the left atrial appendage.

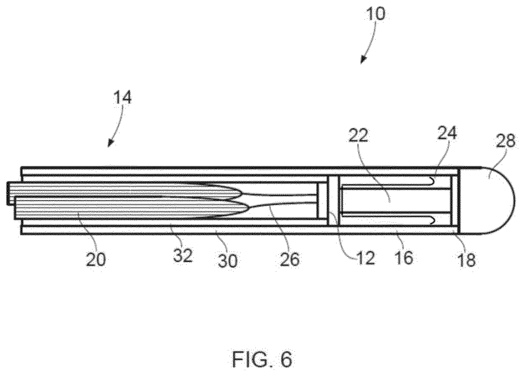

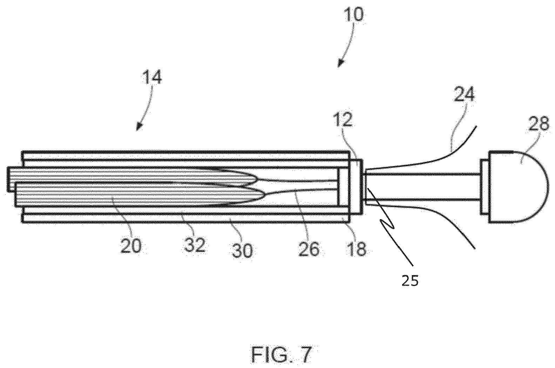

The occlusion device 12 includes a body 22. Any portion of the body 22 may be made from any number of suitable biocompatible materials, e.g., stainless steel, nickel titanium alloys such as Nitinol^{™}, cobalt chromium alloys such as MP35N, other alloys such as ELGILOY^{®} (Elgin, Ill.), various polymers, pyrolytic carbon, silicone, polytetrafluoroethylene (PTFE), or any number of other materials or combination of materials. The body 22 has at least one anchor 24 connected thereto. In some embodiments, the occlusion device 12 includes two anchors. In alternative embodiments, any suitable number of anchors may be provided, such as one, three, four or any other number of anchors. Each anchor 24 is configured to anchor the occlusion device 12 in the left atrial appendage LAA in use. In the illustrated embodiment, the anchor 24 may be welded to the body 22, but it will be appreciated that any suitable arrangement may be used. In alternative embodiments, for example as illustrated in Figures 6 and 7, the anchor 24 may be attached to the body 22 via a releasable fastener 25. The releasable fastener 25 may be a circular or split ring configured to releasably secure the anchor 24 to the body 22. In some embodiments, the circular or split ring 25 may be received in a corresponding groove on the body 22. It will be appreciated that the circular or split ring 25 and groove may be configured to mount the anchor 24 to the body 22 via a snap-fit arrangement.

The occlusion assembly 20 is connected to the body 22. In the arrangement shown, the occlusion assembly 20 is connected to the body 22 by at least one connecting member 26, for example a plurality of connecting members 26. However, it will be appreciated that any suitable connection arrangement may be used, for example the occlusion assembly 20 may be directly connected to the body 22 in some arrangements. The occlusion device 12 is arranged such that the at least one anchor 24 is positioned distally relative to the occlusion assembly 20. Put another way, the anchor 24 is distal and longitudinally adjacent to the occlusion assembly 20.

As discussed above, the occlusion device 12 is at least partially positioned within the catheter body 16. In the arrangement shown, the body 22 includes a distal head portion 28. In the embodiment shown, the head portion 28 protrudes from the distal tip 18 of the catheter body 16 when the occlusion device 12 is in the delivery configuration. Put another way, when the occlusion device 12 is in the delivery configuration, the head portion 28 protrudes from the catheter body 16 and the rest of the occlusion device is positioned proximal to the head portion 28 and is positioned within the catheter body 16. The head portion 28 defines a distal end surface that is curved, for example continuously curved.

The head portion 28 defines an atraumatic tip of the catheter system 10. The atraumatic tip may be positioned at the distal end portion 18 of the catheter body 16. The atraumatic tip may be used to facilitate the advancement of the catheter system 10 through the patient's skin and vasculature. The atraumatic tip may be configured so as to prevent intravascular trauma during delivery of the catheter system 10 to the native valve annulus. In some embodiments, the atraumatic tip may be a flexible curved or tapered tip (i.e. a flexible curved or tapered distal end face). In one embodiment, the atraumatic tip may have a distal opening for accommodating a guidewire (not shown). It will be understood that the tip and/or curvature of the atraumatic tip may be varied depending upon the particular sizing/configuration as required.

In some embodiments, the catheter body 16 includes an outer shaft 30 and an inner shaft 32 disposed within the outer shaft 30. In such embodiments, the body 22 may be positioned so as to protrude from a distal end of the inner shaft 32 so as to be positioned within the outer shaft 30 only. The occlusion assembly 20 may be positioned within the inner shaft 32.

In this embodiment, at least a part of the head portion 28 is oversized relative to inner shaft 32 such that at least a part of the head portion 28 cannot be positioned within the inner shaft 32. This arrangement maintains the position of the occlusion device 12 relative to the distal tip 18 of the inner shaft 30 of the catheter body 16, which has been found to facilitate positioning of the occlusion device 12 in the left atrial appendage LAA. The head portion 28 may define an outer diameter that is substantially the same as the outer diameter of the outer shaft 30. The head portion 28 may define a reduced diameter region that is received in the distal end of the inner shaft 32. It will be appreciated that in some embodiments, the head portion 28 may be omitted. In such arrangements, substantially all of the occlusion device 12 may be positioned within the catheter body 16 (e.g. within the inner shaft 32) when the occlusion device 12 is in the delivery configuration.

Initially, the inner shaft 32 is arranged such that the distal end of the inner shaft bears against the body 22 of the occlusion device 12. The anchors 24 and the head portion 28 are arranged distally to the inner shaft 32. In this initial configuration, the outer shaft 30 extends over the body 22 of the occlusion device 12 and bears against the head portion 28.

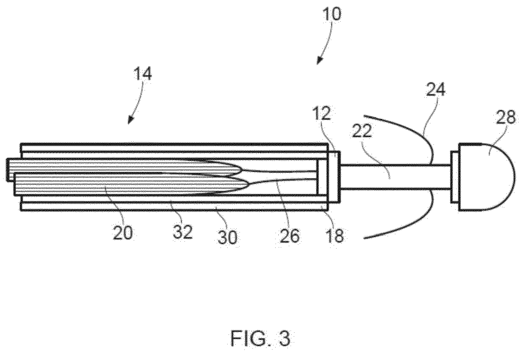

Partial deployment of the occlusion device 12 from the catheter body 16 is achieved by relative movement between the catheter body 16 and the occlusion device 12. This relative movement between the catheter body 16 and the occlusion device may be achieved by a proximal retraction of the catheter body 16. In the embodiment shown, this partial deployment of the occlusion device 12 is carried out via a proximal retraction of the outer shaft 30. This proximal retraction of the outer shaft 30 releases the anchors 24, as is illustrated in Figure 3. In this configuration, the occlusion assembly 20 remains in the delivery configuration. In the deployed configuration, the anchor 24 is configured to anchor the occlusion device 12 in the left atrial appendage LAA. In alternative embodiments, it will be appreciated that this partial deployment of the occlusion device 12 to release the anchors 24 may be achieved by a distal extension of the occlusion device 12 relative to the catheter body 16, for example via a hydraulic deployment mechanism or via a distal extension of the inner shaft 32.

Once the anchors 24 have been released, the catheter body 16 (for example the inner and outer shafts 30, 32) may be proximally retracted. In order to control the catheter body 16 (for example the inner and outer shafts 30, 32), a manipulator may be attached thereto which is operable by a physician. It will be understood that in embodiments including the inner and outer shafts 30, 32 a separate manipulator may be connected to each of the inner and outer shafts 30, 32. Alternatively, a single manipulator may be connected to both of the inner and outer shafts 30, 32 to control them simultaneously.

The at least one anchor may be configured to be self-expanding. Put another way, the at least one anchor 24 may be formed from a self-expanding material. The anchor 24 may move from the delivery configuration to the deployed configuration via one or more shape memory portions of the anchor 24. The anchor may be at least partially formed from a shape memory material, for example a shape memory alloy or shape memory polymer. In the embodiments discussed herein, the shape memory material is nitinol, but it will be appreciated that any suitable shape memory material may be used. In some embodiments, the anchor 24 may move from the delivery configuration to the deployed configuration via any other suitable arrangement such as a biasing arrangement, or via an actuating means. In embodiments include the circular or split ring 25 as illustrated in Figures 6 and 7, the circular or split ring 25 may be formed from the same material as the anchor 24. Put another way, in some embodiments, the circular or split ring 25 may be integrally formed with the anchor arms 24. In alternative embodiments, the circular or split ring 25 may be formed from a different material to the anchor arms 24 and may be connected thereto, e.g. via welding.

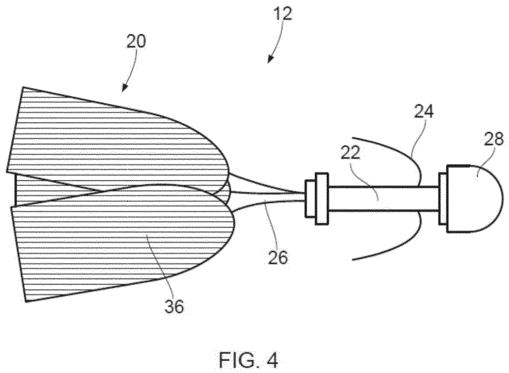

Referring to Figure 4, the occlusion device 12 is shown in a deployed configuration. In some embodiments, deployment of the occlusion device 12 from the catheter system may be achieved by a proximal retraction of the catheter body 16 (for example the outer shaft 30 and the inner shaft 32, in this embodiment). This proximal retraction of the catheter body 16 enables the occlusion assembly 20 to move from the radially compressed configuration to the deployed, or radially expanded configuration. In alternative embodiments, the relative movement between the catheter body 16 and the occlusion device 12 may be achieved by a distal extension of the occlusion device 12 relative to the catheter body 16, for example via a hydraulic deployment mechanism.

The occlusion assembly 20 is formed from a plurality of elongate occluding filaments 36. The occluding filaments extend in a direction away from the connecting member or the body 22. Put another way, the occluding filaments 36 extend in a direction away from the at least one anchor 24. Each of the plurality of elongate occluding filaments 36 is configured to move into a radially expanded configuration upon deployment from the catheter body 16. The plurality of elongate occluding filaments 36 is configured to occlude and seal the left atrial appendage LAA in the radially expanded configuration.

Once the catheter body 16 is in place adjacent the left atrial appendage LAA, the occlusion device 12 is deployed from the catheter body as has been described above. The occlusion device 12 is first partially deployed from the catheter body 16 to enable the anchor 24 to move from the delivery configuration to the deployed configuration to anchor the occlusion device in the left atrial appendage LAA, whilst the occlusion assembly is retained in the delivery configuration.

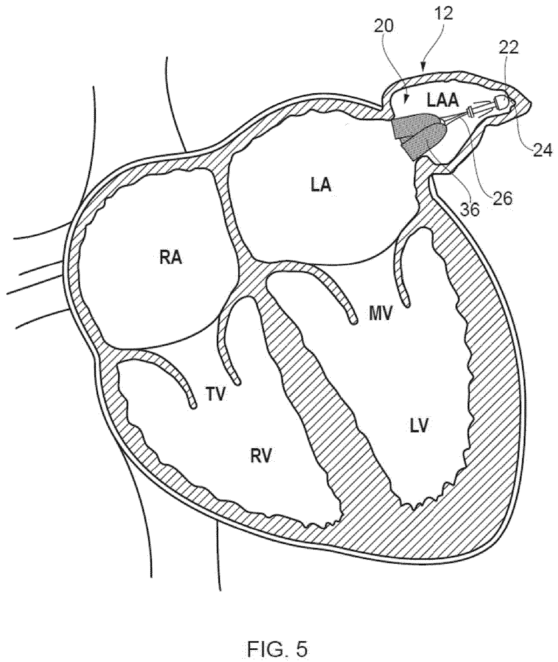

Referring to Figure 5, the occlusion device 12 is deployed from the catheter body 16 to enable the occlusion assembly 20 to move from the radially compressed configuration to the deployed, or radially expanded configuration, to occlude the left atrial appendage LAA. It will be understood that the occlusion device 12 seals against the left atrial appendage LAA to prevent a thrombus from passing therethrough. Once the occlusion device 12 has been deployed, the catheter body 16 is removed and occlusion device 12 is left in the left atrial appendage LAA.

An atrial facing portion of the occlusion device 12 (e.g. the atrial side of the occlusion assembly 20) may be configured to prevent or impede the formation of a thrombus thereon. In some embodiments, the one or more, for example all or substantially all, of the elongate filaments 36 may define a surface that is configured to prevent or impede the formation of a thrombus thereon. The surface of the elongate filaments 36 may be coated with an anti-thrombogenesis film or coating, may be heparinized, and/or may include a surface structure which impedes thrombogenesis, repels blood media and/or is inert to blood media. It will be appreciated that in such embodiments, one or more of the elongate filaments 36 of the occlusion assembly 20 may be configured to prevent or impede the formation of a thrombus thereon. It will be understood that the one or more of the elongate filaments 36 may be partially (e.g. at the atrial side region) or entirely configured to prevent or impede the formation of a thrombus thereon.

The plurality of elongate occluding filaments 36 may be arranged in a bushy configuration or a cluster configuration. According to the invention, in the radially expanded configuration, the occlusion assembly 20 is configured to be oversized relative to the opening of the left atrial appendage LAA. It will be understood that each of the plurality of elongate occluding filaments 36 may be configured to be radially compressible in the radially expanded configuration. The plurality of elongate occluding filaments 36 of the occlusion assembly 20 may be configured and arranged to inter-engage and/or inter-mesh upon application of an external force to prevent a thrombus from passing therethrough, in use. It will be appreciated that due to the occlusion assembly 20 being oversized relative to the opening of the left atrial appendage LAA, the left atrial appendage LAA will apply an external force to the plurality of elongate occluding filaments 36 to cause them to inter-engage and/or inter-mesh to occlude the left atrial appendage LAA.

Each of the plurality of elongate occluding filaments 36 may be configured to swell and/or radially expand to move into the radially expanded configuration. The plurality of elongate occluding filaments 36 may move from the radially compressed configuration to the radially expanded configuration via one or more shape memory portions thereof. Each of the plurality of elongate occluding filaments 36 may be at least partially formed from a shape memory material, for example a shape memory alloy or shape memory polymer. In the embodiments discussed herein, the shape memory material is nitinol, but it will be appreciated that any suitable shape memory material may be used. In some embodiments, each of the plurality of elongate occluding filaments 36 may move from the delivery configuration to the deployed configuration via any other suitable arrangement, for example each of the plurality of elongate occluding filaments 36 may be formed from a sponge material that is configured to swell upon deployment from the catheter body 16.

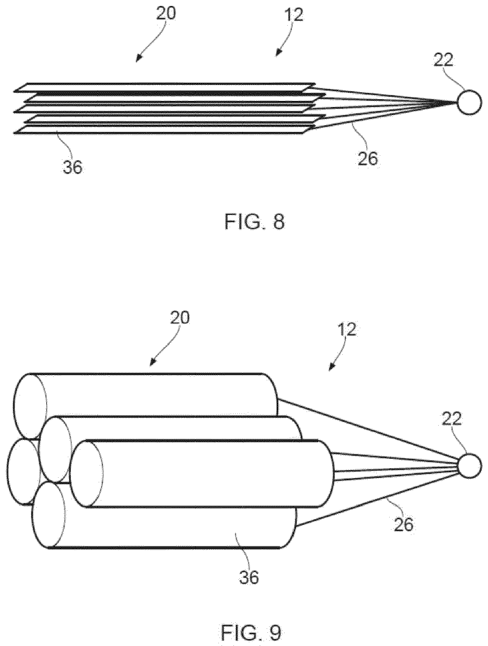

Each of the plurality of elongate occluding filaments 36 may be fingers. The plurality of elongate occluding filaments 36 may be provided in the form of elongate strips, as illustrated in Figure 8. The plurality of elongate occluding filaments 36 may be connected to the body 22, for example via one or more connecting members 26. In some embodiments, a single connecting member 26 may connect a plurality of occluding filaments 36 to the body 22. In other embodiments, a single connecting member 26 may connect a single occluding filament 36 to the body 22. In some embodiments, when in the radially expanded configuration, the plurality of elongate occluding filaments 36 may move into a substantially cylindrical or tubular shape, as is shown in Figure 9. Although not illustrated, in alternative embodiments, the plurality of elongate occluding filaments 36 may move into substantially spiral filaments or conical filaments, or any other suitable radially expanded shape. It will be understood that a combination of differently shaped radially expanded occluding filaments may be provided on the occlusion device 12. In some embodiments, the occlusion assembly 20 may include a core and a plurality of elongate occluding filaments 36 arranged around said core. It will be appreciated that the core may be formed from a self-expanding material, for example a shape memory material such as a shape memory polymer or nitinol. The core may be substantially rigid.

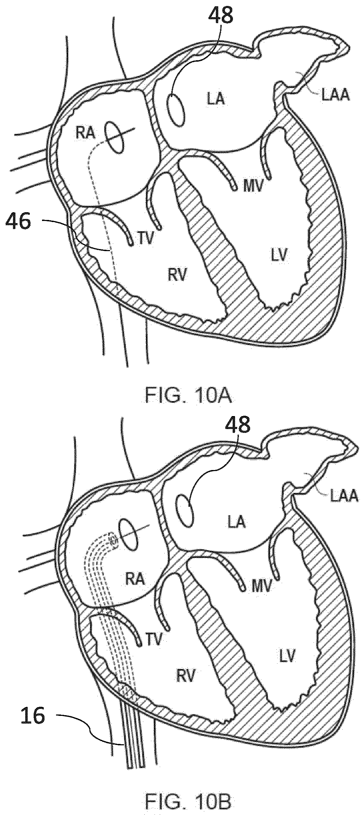

Referring to Figures 10A to 10D, a method for delivering and deploying an occlusion device to a left atrial appendage LAA will now be described.

In an exemplary embodiment, a guidewire 46 is advanced after having been introduced into the vasculature via a percutaneous entry point and tracked through the vasculature into a left atrium LA of a heart. Intravascular access to the right atrium RA may be achieved via a percutaneous access site to femoral venous access up to the inferior venal cava, or other known access routes. Thereafter, a guidewire is advanced through the circulatory system, eventually arriving at the heart.

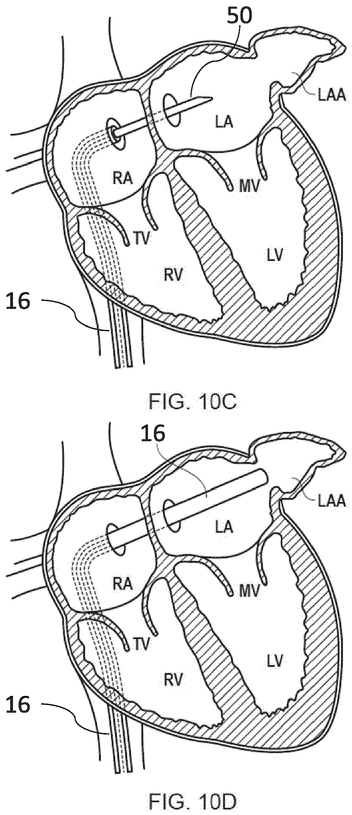

The guidewire 46 is directed into the right atrium RA (see Figure 10A), traverses the right atrium and is made to traverse, with the aid of a pre-existing hole 48 (see Figure 10B) or a transseptal needle 50 (see Figure 10C) an atrial septum, thereby entering the left atrium LA. Once the guidewire is positioned, the entry port and the atrial septum are dilated to permit entry of the catheter body 16 of the catheter system 10 into the left atrium LA towards the left atrial appendage LAA (see Figure 10D).

Thereafter, the occlusion device 12 is advanced through the catheter body 16 into the left atrium LA and is positioned proximate the left atrial appendage LAA. Although described as a transfemoral antegrade approach for percutaneously accessing left atrium LA, the catheter body 16 may be positioned within the desired area of the heart via different methods or routes. For example, and not by way of limitation, another possible path would be through the radial vein into the brachial vein, through the subclavian vein, through the superior vena cava into the right atrium, and then transseptally into the left atrium. Yet another possible path would be through the femoral artery into the aorta, through the aortic valve into the left ventricle, and then retrograde through the mitral valve into the left atrium. In another embodiment, the left ventricle LV may be accessed via a transapical approach, and the catheter body 16 may be advanced through the left ventricle LV, the mitral valve, and into the left atrium LA adjacent the left atrial appendage LAA. Yet another possible path would be to use a transjugular approach to the left atrial appendage LAA. In addition, although described with the use of a guidewire, in another embodiment hereof the catheter body 16 may access the left atrium LA without the use of a guidewire.

Once the catheter body 16 is in place adjacent the left atrial appendage LAA, the occlusion device 12 is deployed from the catheter body as has been described above. The occlusion device 12 is first partially deployed from the catheter body 16 to enable the anchor to move from the delivery configuration to the deployed configuration to anchor the occlusion device in the left atrial appendage LAA. Put another way, the occlusion device 12 is anchored in the left atrial appendage LAA. The occlusion device 12 is then further, e.g. fully, deployed from the catheter body to enable the occlusion assembly 20 to move from the radially compressed configuration to the deployed, or radially expanded configuration, to occlude the left atrial appendage LAA, as is shown in Figure 5, and seal against the left atrial appendage LAA and to prevent a thrombus from passing therethrough. Once the occlusion device 12 has been deployed, the catheter body 16 is removed and occlusion device 12 is left in the left atrial appendage LAA.

An atrial facing portion of the occlusion device 12 (e.g. the atrial side of the occlusion assembly 20) may be configured to prevent or impede the formation of a thrombus thereon. In some embodiments, the one or more, for example all or substantially all, of the elongate filaments 36 may define a surface that is configured to prevent or impede the formation of a thrombus thereon. The surface of the elongate filaments 36 may be coated with an anti-thrombogenesis film or coating, may be heparinized, and/or may include a surface structure which impedes thrombogenesis, repels blood media and/or is inert to blood media. It will be appreciated that in such embodiments, one or more of the elongate filaments 36 of the occlusion assembly 20 may be configured to prevent or impede the formation of a thrombus thereon. It will be understood that the one or more of the elongate filaments 36 may be partially (e.g. at the atrial side region) or entirely configured to prevent or impede the formation of a thrombus thereon.

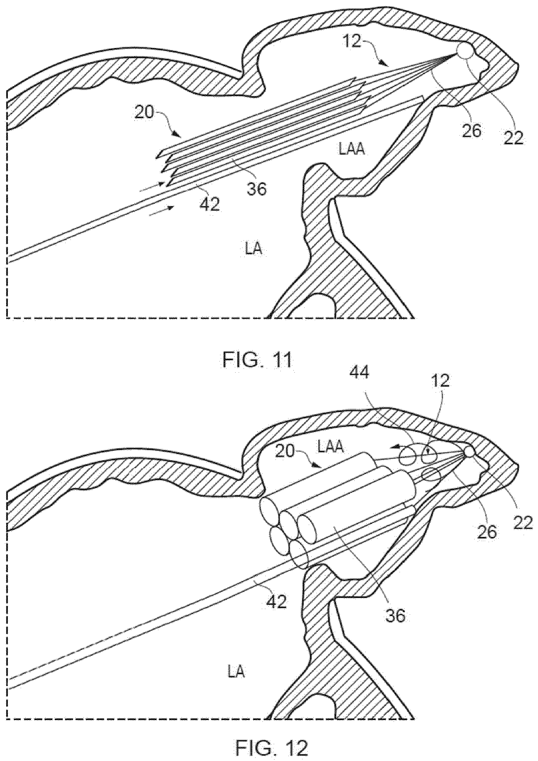

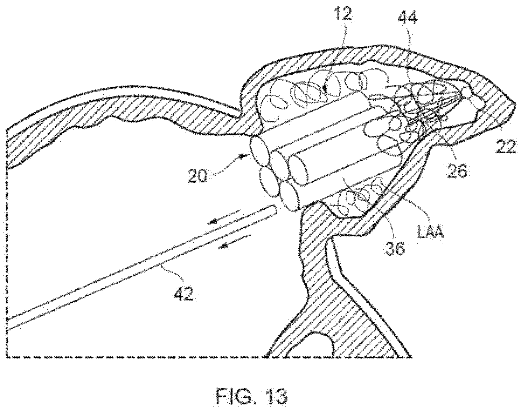

Referring now to Figure 11 to 13, once the catheter body (omitted from the figures for clarity) is in place adjacent the left atrial appendage LAA, the occlusion device 12 is deployed from the catheter body. The occlusion device 12 is anchored in the left atrial appendage LAA. The occlusion device 12 is first partially deployed from the catheter body to enable the anchor to move from the delivery configuration to the deployed configuration to anchor the occlusion device in the left atrial appendage LAA.

The occlusion device 12 is then further, e.g. fully, deployed from the catheter body to enable the occlusion assembly 20 to move from the radially compressed configuration (shown in Figure 11) to the deployed, or radially expanded configuration (shown in Figure 12) to occlude the left atrial appendage LAA and seal against the left atrial appendage LAA and to prevent a thrombus from passing therethrough.

In some embodiments, for example those illustrated in Figures 11 to 13, the catheter system 10 may include a delivery member 42 configured and arranged to deliver a filler material 44, for example biogel, to a position distal to the occlusion assembly 20 in the left atrial appendage LAA. The delivery member 42 may be positioned within the catheter body 16. The delivery member 42 may be arranged to extend through or adjacent to the plurality of elongate filaments 36. In alternative embodiments, the delivery member may be provided on the body 22, for example on the head portion 28, of the occlusion device. In such arrangements, the body 22 may include an internal chamber containing the filler material and the aperture 40 may be fluidly coupled to the chamber for dispensing the filler material.

In embodiments where the occlusion device 12 or catheter system 10 is configured to deliver a filler material in the left atrial appendage LAA distal to the occlusion assembly 20, the method may include positioning the occlusion device 12 in the left atrial appendage LAA as has been described above. When the anchor 24 has anchored the occlusion device 12 in the left atrial appendage LAA, a filler material may be deposited in the left atrial appendage LAA distal to the occlusion assembly 20, as is shown in Figure 12. In embodiments including a delivery member 42, when the filler material has been deposited, the delivery member 42 is retracted and removed from the left atrial appendage LAA.

Once the occlusion device 12 has been deployed, and the filler material 44 has been deposited, the catheter body 16 is removed and occlusion device 12 is left in the left atrial appendage LAA.

With regard to the terms "distal" and "proximal" within this description, unless otherwise specified, the terms can reference a relative position of the portions of the delivery catheter system with reference to an operator and/or a location in the vasculature or heart. For example, "proximal" can refer to a position closer to the operator of the device or an incision into the vasculature, and "distal" can refer to a position that is more distant from the operator of the device or further from the incision along the vasculature (e.g., the end of the catheter).

## Claims

1. An occlusion device (12) for occluding a left atrial appendage, the device comprising:
a body (22);
at least one anchor (24) connected to the body and moveable between a delivery configuration and a deployed configuration in which the at least one anchor is configured to anchor the occlusion device in the left atrial appendage; and
an occlusion assembly (20) connected to the body,
wherein the occlusion assembly comprises a plurality of elongate occluding filaments (36) extending in a direction away from the at least one anchor, wherein the plurality of elongate occluding filaments are arranged in a cluster configuration, and
wherein each of the plurality of elongate occluding filaments is configured to move from a radially compressed configuration into a radially expanded configuration for occluding and sealing the left atrial appendage,
wherein, in the radially expanded configuration, the occlusion assembly is configured to be oversized relative to an opening of the left atrial appendage, **characterized in that** due to the occlusion assembly being oversized relative to the opening of the left atrial appendage, the left atrial appendage will apply an external force to the plurality of elongate occluding filaments to cause the plurality of elongate occluding filaments to inter-engage and/or inter-mesh to occlude the left atrial appendage.

2. The occlusion device according to claim 1, wherein the plurality of elongate occluding filaments are configured and arranged to inter-engage and/or inter-mesh upon application of the external force for preventing a thrombus from passing therethrough.

3. The occlusion device according to any preceding claim, wherein each of the plurality of elongate occluding filaments are configured to swell and/or radially expand to move into the radially expanded configuration.

4. The occlusion device according to any preceding claim, wherein each of the plurality of elongate occluding filaments are configured to be radially compressible in the radially expanded configuration.

5. The occlusion device according to any preceding claim, wherein each of the plurality of elongate occluding filaments comprises a self-expanding material.

6. The occlusion device according to any preceding claim, wherein the occlusion assembly comprises a core and wherein the plurality of elongate occluding filaments are arranged around said core; and optionally wherein the core is formed from a self-expanding material.

7. The occlusion device according to any preceding claim, wherein the at least one anchor is configured to move into the deployed configuration upon deployment from a catheter body (16).

8. The occlusion device according to any preceding claim, wherein the at least one anchor is formed from a self-expanding material.

9. The occlusion device according to any preceding claim, comprising an actuating arrangement to move the at least one anchor into the deployed configuration.

10. The occlusion device according to any preceding claim, wherein the body comprises a chamber containing a filler material and an aperture (40) fluidly coupled to the chamber for dispensing the filler material; and/or wherein the anchor is connected to the body via a circular or split ring (25).

11. A delivery catheter system (10) comprising:
a catheter assembly (14) comprising an elongate catheter body (16) having a distal tip portion; and
an occlusion device (12) according to claim 1 positioned within the catheter body, wherein the occlusion assembly is arranged such that the at least one anchor is positioned distally relative to the occlusion assembly,
wherein the occlusion device is deployable from the distal tip portion of the catheter body so as to enable the at least one anchor to move from the delivery configuration to the deployed configuration, and
wherein the plurality of elongate occluding filaments are configured to move from the radially compressed configuration to the radially expanded configuration upon deployment from the catheter body.

12. The delivery catheter assembly according to claim 11, comprising a delivery member (42) configured and arranged to deliver a filler material to a position distal to the occlusion assembly.

13. The delivery catheter assembly according to claim 12, wherein the delivery member is positioned within the catheter body; and/or wherein the delivery member is arranged to extend through or adjacent to the plurality of elongate filaments.

## Patentansprüche

1. Okklusionsvorrichtung (12) zum Okkludieren eines linken Herzohrs, die Vorrichtung umfassend: einen Körper (22);
mindestens einen Anker (24), der mit dem Körper verbunden und zwischen einer Zuführkonfiguration und einer entfalteten Konfiguration bewegbar ist, in der der mindestens eine Anker konfiguriert ist, um die Okklusionsvorrichtung in dem linken Herzohr zu verankern; und
eine Okklusionsbaugruppe (20), die mit dem Körper verbunden ist,
wobei die Okklusionsbaugruppe eine Vielzahl von länglichen okkludierenden Filamenten (36) umfasst, die sich in einer Richtung weg von dem mindestens einen Anker erstrecken, wobei die Vielzahl von länglichen okkludierenden Filamenten in einer Clusterkonfiguration angeordnet ist, und
wobei jedes der Vielzahl von länglichen okkludierenden Filamenten konfiguriert ist, um sich aus einer radial komprimierten Konfiguration in eine radial expandierte Konfiguration zum Okkludieren und Abdichten des linken Herzohrs zu bewegen,
wobei, in der radial expandierten Konfiguration, die Okklusionsbaugruppe konfiguriert ist, um relativ zu einer Öffnung des linken Herzohrs überdimensioniert zu sein, **dadurch gekennzeichnet, dass** aufgrund dessen, dass die Okklusionsbaugruppe relativ zu der Öffnung des linken Herzohrs überdimensioniert ist, das linke Herzohr eine externe Kraft auf die Vielzahl von länglichen okkludierenden Filamenten ausübt, um zu bewirken, dass die Vielzahl von länglichen okkludierenden Filamenten ineinandergreifen und/oder sich vermaschen, um das linke Herzohr zu okkludieren.

2. Okklusionsvorrichtung nach Anspruch 1, wobei die Vielzahl von länglichen okkludierenden Filamenten konfiguriert und angeordnet ist, um bei dem Ausüben der externen Kraft ineinanderzugreifen und/oder sich zu vermaschen, zum Verhindern, dass ein Thrombus dort hindurchtritt.

3. Okklusionsvorrichtung nach einem der vorstehenden Ansprüche, wobei jedes der Vielzahl von länglichen okkludierenden Filamenten konfiguriert ist, um anzuschwellen und/oder radial zu expandieren, um sich in die radial expandierte Konfiguration zu bewegen.

4. Okklusionsvorrichtung nach einem der vorstehenden Ansprüche, wobei jedes der Vielzahl von länglichen okkludierenden Filamenten konfiguriert ist, um in der radial expandierten Konfiguration radial komprimierbar zu sein.

5. Okklusionsvorrichtung nach einem der vorstehenden Ansprüche, wobei jedes der Vielzahl von länglichen okkludierenden Filamenten ein selbstexpandierendes Material umfasst.

6. Okklusionsvorrichtung nach einem der vorstehenden Ansprüche, wobei die Okklusionsbaugruppe einen Kern umfasst und wobei die Vielzahl von länglichen okkludierenden Filamenten um den Kern herum angeordnet sind; und optional wobei der Kern aus einem selbstexpandierenden Material ausgebildet ist.

7. Okklusionsvorrichtung nach einem der vorstehenden Ansprüche, wobei der mindestens eine Anker konfiguriert ist, um sich in die entfaltete Konfiguration beim Entfalten aus einem Katheterkörper (16) zu bewegen.

8. Okklusionsvorrichtung nach einem der vorstehenden Ansprüche, wobei der mindestens eine Anker aus einem selbstexpandierenden Material ausgebildet ist.

9. Okklusionsvorrichtung nach einem der vorstehenden Ansprüche, umfassend eine Betätigungsanordnung, um den mindestens einen Anker in die entfaltete Konfiguration zu bewegen.

10. Okklusionsvorrichtung nach einem der vorstehenden Ansprüche, wobei der Körper eine Kammer, die ein Füllmaterial enthält, und einen Durchlass (40) umfasst, der an die Kammer zum Abgeben des Füllmaterials fluidisch gekoppelt ist; und/oder wobei der Anker mit dem Körper über einen kreisförmigen oder geteilten Ring (25) verbunden ist.

11. Zuführkathetersystem (10), umfassend:
eine Katheterbaugruppe (14), umfassend einen länglichen Katheterkörper (16), der einen distalen Spitzenabschnitt aufweist; und
eine Okklusionsvorrichtung (12) nach Anspruch 1, die innerhalb des Katheterkörpers positioniert ist, wobei die Okklusionsbaugruppe derart angeordnet ist, dass der mindestens eine Anker distal relativ zu der Okklusionsbaugruppe positioniert ist,
wobei die Okklusionsvorrichtung aus dem distalen Spitzenabschnitt des Katheterkörpers entfaltbar ist, um dem mindestens einen Anker zu ermöglichen, sich aus der Zuführkonfiguration in die entfaltete Konfiguration zu bewegen, und
wobei die Vielzahl von länglichen okkludierenden Filamenten konfiguriert ist, um sich aus der radial komprimierten Konfiguration in die radial expandierte Konfiguration beim Entfalten aus dem Katheterkörper zu bewegen.

12. Zuführkatheterbaugruppe nach Anspruch 11, umfassend ein Zuführelement (42), das konfiguriert und angeordnet ist, um ein Füllmaterial an eine Position distal zu der Okklusionsbaugruppe zuzuführen.

13. Zuführkatheterbaugruppe nach Anspruch 12, wobei das Zuführelement innerhalb des Katheterkörpers positioniert ist; und/oder wobei das Zuführelement angeordnet ist, um sich durch die oder benachbart zu der Vielzahl von länglichen Filamenten zu erstrecken.

## Revendications

1. Dispositif d'occlusion (12) permettant d'occlure un appendice auriculaire gauche, le dispositif comprenant : un corps (22) ;
au moins un ancrage (24) relié au corps et mobile entre une configuration de pose et une configuration expansée dans laquelle l'au moins un ancrage est configuré pour ancrer le dispositif d'occlusion dans l'appendice auriculaire gauche ; et
un ensemble d'occlusion (20) relié au corps,
dans lequel l'ensemble d'occlusion comprend une pluralité de filaments d'occlusion allongés (36) s'étendant dans une direction s'éloignant de l'au moins un ancrage, dans lequel la pluralité de filaments d'occlusion allongés sont agencés selon une configuration en faisceau, et
dans lequel chaque filament de la pluralité de filaments d'occlusion allongés est configuré pour passer d'une configuration comprimée radialement à une configuration expansée radialement pour occlure et sceller l'appendice auriculaire gauche,
dans lequel, dans la configuration radialement expansée, l'ensemble d'occlusion est configuré pour être surdimensionné par rapport à une ouverture de l'appendice auriculaire gauche, **caractérisé en ce que** du fait que l'ensemble d'occlusion est surdimensionné par rapport à l'ouverture de l'appendice auriculaire gauche, l'appendice auriculaire gauche appliquera une force externe à la pluralité de filaments d'occlusion allongés pour amener la pluralité de filaments d'occlusion allongés à venir en prise mutuelle et/ou à s'entremêler pour occlure l'appendice auriculaire gauche.

2. Dispositif d'occlusion selon la revendication 1, dans lequel la pluralité de filaments d'occlusion allongés sont configurés et agencés pour venir en prise mutuelle et/ou s'entremêler lors de l'application de la force externe pour empêcher un thrombus de passer à travers ceux-ci.

3. Dispositif d'occlusion selon l'une quelconque revendication précédente, dans lequel chaque filament de la pluralité de filaments d'occlusion allongés est configuré pour gonfler et/ou se déployer radialement pour passer dans la configuration radialement expansée.

4. Dispositif d'occlusion selon l'une quelconque revendication précédente, dans lequel chaque filament de la pluralité de filaments d'occlusion allongés est configuré pour être radialement compressible dans la configuration radialement expansée.

5. Dispositif d'occlusion selon l'une quelconque revendication précédente, dans lequel chaque filament de la pluralité de filaments d'occlusion allongés comprend un matériau autoexpansible.

6. Dispositif d'occlusion selon l'une quelconque revendication précédente, dans lequel l'ensemble d'occlusion comprend une âme et dans lequel la pluralité de filaments d'occlusion allongés sont agencés autour de ladite âme ; et éventuellement dans lequel l'âme est formée à partir d'un matériau autoexpansible.

7. Dispositif d'occlusion selon l'une quelconque revendication précédente, dans lequel l'au moins un ancrage est configuré pour passer dans la configuration déployée lors d'un déploiement à partir d'un corps de cathéter (16).

8. Dispositif d'occlusion selon l'une quelconque revendication précédente, dans lequel l'au moins un ancrage est formé d'un matériau autoexpansible.

9. Dispositif d'occlusion selon l'une quelconque revendication précédente, comprenant un agencement d'actionnement pour déplacer l'au moins un ancrage dans la configuration déployée.

10. Dispositif d'occlusion selon l'une quelconque revendication précédente, dans lequel le corps comprend une chambre contenant un matériau de remplissage et une ouverture (40) accouplée fluidiquement à la chambre pour distribuer le matériau de remplissage ; et/ou dans lequel l'ancrage est relié au corps par l'intermédiaire d'un anneau circulaire ou fendu (25).

11. Système de pose de cathéter (10) comprenant :
un ensemble cathéter (14) comprenant un corps de cathéter allongé (16) ayant une partie de pointe distale ; et
un dispositif d'occlusion (12) selon la revendication 1 positionné à l'intérieur du corps de cathéter, dans lequel l'ensemble d'occlusion est agencé de telle sorte que l'au moins un ancrage est positionné de manière distale par rapport à l'ensemble d'occlusion,
dans lequel le dispositif d'occlusion est déployable à partir de la partie de pointe distale du corps de cathéter de manière à permettre à l'au moins un ancrage de passer de la configuration de pose à la configuration déployée, et
dans lequel la pluralité de filaments d'occlusion allongés sont configurés pour passer de la configuration radialement comprimée à la configuration radialement expansée lors du déploiement depuis le corps de cathéter.

12. Ensemble cathéter de pose selon la revendication 11, comprenant un élément de pose (42) configuré et agencé pour acheminer un matériau de remplissage en une position distale par rapport à l'ensemble d'occlusion.

13. Ensemble cathéter de pose selon la revendication 12, dans lequel l'élément de pose est positionné à l'intérieur du corps de cathéter ; et/ou dans lequel l'élément de pose est agencé pour s'étendre à travers ou de manière adjacente à la pluralité de filaments allongés.
